# EUROPÄISCHE PATENTSCHRIFT

(11) **EP 0 435 226 B1**
(45) Veröffentlichungstag und Bekanntmachung des Hinweises auf die Patenterteilung: **24.05.1995**
(21) Anmeldenummer: 90125282.5
(22) Anmeldetag: 21.12.1990
(51) Int. Cl.: C12Q 1/02, C12Q 1/10, C12Q 1/14, G01N 33/48

(54) **Phagozytose-Test**
Phagocytosis assay
Essai de phagocytose

(30) Priorität: 29.12.1989 DE 3943325; 04.05.1990 DE 4014393
(43) Veröffentlichungstag der Anmeldung: 03.07.1991
(73) Patentinhaber: Orpegen Medizinisch-Molekularbiologische Forschungsgesellschaft m.b.H., 69115 Heidelberg (DE)
(72) Erfinder: Nebe, Thomas C., Dr., W-6802 Ladenburg (DE)
(74) Vertreter: Huber, Bernhard, Dipl.-Chem.

(56) Entgegenhaltungen:
- JOURNAL OF IMMUNOLOGICAL METHODS, Band 88, Nr. 2, April 1986, Amsterdam (NL); T. ODA et al., Seiten 175-183#
- BIOLOGICAL ABSTRACTS, Band 77, Nr. 11, 1984, Philadelphia, PA (US); C.-F. BASSOE et al., Seite 9322, Nr. 84421#
- CHEMICAL ABSTRACTS, Band 98, Nr. 7, 14 Februar 1983, Columbus, OH (US); R.C. ALLEN, Seite 494, Nr. 51641r#
- CHEMICAL ABSTRACTS, Band 94, Nr. 11, 16 März 1981, Columbus, OH (US); M. NAKAMURA et al., Seite 537, Nr. 81876f#
- BIOLOGICAL ABSTRACTS, Band 78, Nr. 2, 1984; C.-F. BASSOE, Seiten 1301-1302, Nr. 11447#
- C.M. HÄNSCH, "Labor und Diagnose", Auflage 3: "Granulozyten-Funktionsprüfung",1988, Medizinische Verlagsgesellschaft, Marburg (DE); Seiten 798-806#

## Beschreibung

Die Erfindung betrifft ein Verfahren zur Bestimmung der Phagozytose-Aktivität von Säugerleukozyten sowie ein dazu geeignetes Reagenzien-Kit.

Der Säugerorganismus verfügt zur Abwehr von eindringenden Mikroorganismen sowie zur Beseitigung von alten und beschädigten Zellen oder Zelltrümmern über Mechanismen zur Auflösung dieser unerwünschten Bestandteile. Diese unerwünschten Bestandteile werden an der Oberfläche der zur Phagozytose befähigten Leukozyten im peripheren Blut, im wesentlichen Monozyten, Makrophagen und polymorphkernige Leukozyten, gebunden. Nur wenn diese zur Phagozytose spezialisierte Zelle den gebundenen Bestandteil als "unerwünscht" erkennt, wird der Bestandteil von der Zelle umschlossen und dann lysiert. Erkannt werden v. a. solche Bestandteile, die opsoniert wurden, d.h. an deren Oberfläche Opsonine, z.B. Antikörper und Komplement, gebunden sind. Eine Störung der Phagozytose kann also sowohl verursacht werden durch fehlende Opsonierfähigkeit als auch durch mangelnde Phagozytose-Aktivität. Wenn diese Funktionen gestört sind, so führt dies zu einer Schwächung des Immunsystems und zu einer stärkeren Anfälligkeit für Infektionen.

Es ist nun wichtig für die klinische Diagnostik, bei Vorliegen einer Immunschwäche festzustellen, inwieweit Opsonierfähigkeit und Phagozytose-Aktivität beeinträchtigt sind. Zum Nachweis der Phagozytose-Aktivität sind bereits verschiedene Verfahren bekannt, bei denen man zu isolierten Granulozyten mit fluoreszierenden Substanzen markierte Teilchen oder Mikroorganismen zugibt und anschließend die aufgenommene Fluoreszenz mißt. Die bekannten Verfahren befriedigen jedoch noch nicht, da sie einerseits aufwendig sind, nicht mit Vollblut durchgeführt werden können und außerdem nicht reproduzierbare Ergebnisse liefern. Ergebnisse, die zu verschiedenen Zeiten mit verschiedenen Präparationen erhalten wurden, sind nicht vergleichbar. So ist beispielsweise aus Journal of Immunological Methods, 88 (1986) 175-183 ein Verfahren zur Bestimmung der Phagozytose bekannt, bei dem aus menschlichem Blut die polymorphkernigen Leukozyten isoliert werden und mit Fluoreszenz-markierten Latexteilchen bzw. Fluoreszenz-markierten Mikroorganismen und Hefen inkubiert wurden. Um extrazellulär oder adsorptiv gebundene Mikroorganismen unterscheiden zu können, wird Methylenblau zugegeben, das die Fluoreszenz quencht. Auf diese Weise konnten die intrazellulär aufgenommenen fluoreszierenden Organismen gut nachgewiesen werden. Nachteil dieses Verfahrens ist es jedoch, daß eine Abtrennung der Zellen erfolgen muß und daß die Genauigkeit nicht ausreichend ist.

In Biological Abstracts, 77, 11, 1984, S. 9322 wird ein Verfahren zur Bestimmung der Phagozytoseaktivität von isoliertem Leukozyten beschrieben, bei dem unter Schütteln bei 37°C/15 Minuten mit fluoreszenz-markierten Bakterien inkubiert wird, wobei die Phagozytose durch Abkühlen gestoppt wird. Dann wird die Konzentration von polymorphkernigen Leukozyten durch Zugabe von Acridinorange bestimmt. Dieses Verfahren eignet sich jedoch nicht zur Bestimmung der Phagozytoseaktivität direkt in Körperflüssigkeiten, insbesondere in Vollblut.

Aufgabe der vorliegenden Erfindung war es daher, ein einfaches Verfahren zur genauen und reproduzierbaren Bestimmung der Opsonierfähigkeit und Phagozytose-Aktivität in Vollblut zur Verfügung zu stellen.

Diese Aufgabe wird gelöst durch ein Verfahren zur Bestimmung der Phagozytose-Aktivität von Säugerleukozyten, das dadurch gekennzeichnet ist, daß man eine Körperflüssigkeit mit einer definierten Menge von Fluoreszenzmarkierten Bakterien versetzt, unter Schütteln 5 bis 60 Minuten bei einer Temperatur von 30 bis 40°C inkubiert, die Phagozytose durch Abkühlen stoppt, eine quenchende Substanz zugibt, die in der Lösung vorhandenen, nicht phagozytierten Bakterien in ihrer Fluoreszenz unterdrückt, eine Salzlösung zugibt, die die in der Probelösung vorhandenen Erythrozyten lysiert, einen fluoreszierenden DNA-Farbstoff zugibt der ein anderes Emissionsspektrum aufweist als die fluoreszenzmarkierten Bakterien und anschließend die Fluoreszenz in einem Fluoreszenz-Zytometer auswertet.

Überraschenderweise wurde festgestellt, daß bei Einhaltung der oben genau definierten Bedingungen die Phagozytose-Aktivität in einfacher Weise in physiologischer Umgebung nachgewiesen werden kann und standardisiert werden kann, wobei die Genauigkeit durch Ausgrenzung von Aggregations-Artefakten erhöht wurde. Darüberhinaus kann durch Einsatz von nicht vorher opsonierten E. coli die Opsonierfähigkeit festgestellt werden. Somit lassen sich erstmals Unterschiede von Tag zu Tag, zwischen verschiedenen Bakterienarten oder zwischen kranken und gesunden Personen oder durch Medikamenten-Behandlung reproduzierbar untersuchen.

Das erfindungsgemäße Verfahren eignet sich zur Bestimmung der Phagozytose-Aktivität von Säugerleukozyten, insbesondere von Human- und Nagerleukozyten. Der Nachweis erfolgt in einer Körperflüssigkeit, die Leukozyten enthält. Insbesondere wird die Bestimmung in Vollblut oder Synovialflüssigkeit durchgeführt.

Erfindungsgemäß werden zum Nachweis der Phagozytose Bakterien verwendet, die Fluoreszenz-markiert sind. Bevorzugt wird für die Fluoreszenzmarkierung FITC verwendet. Die Markierung mit FITC ist vorteilhaft, da FITC ein definiertes schmales Fluoreszenzspektrum aufweist, was eine Mehrfarben-Fluoreszenzanalyse durch gleichzeitige Markierung mit z.B. Phycoerythrin markierten Antikörpern zuläßt. Außerdem erfolgt die Färbung in einem niedrigen Intensitätsbereich, der eine gleichzeitige Fluoreszenzanalyse mit Farbstoffen benachbarter Emissions-Wellenlänge zuläßt. Die Markierung der Bakterien kann in an sich bekannter Weise geschehen. Für die Untersuchung des Phagozytose-Vorganges können verschiedene Bakterienarten verwendet werden. Geeignet sind u. a. E. coli, Staphylococcus aureus und Pseudomonas aeroginosa. Da verschiedene Patienten offensichtlich unterschiedlich auf verschiedene Bakterien reagieren kann es vorteilhaft sein, die Untersuchung mit verschiedenen Bakterienarten durchzuführen. Bevorzugt wird für das erfindungsgemäße Verfahren E. coli eingesetzt.

In einem bevorzugten Verfahren zur Markierung von Bakterien werden E. coli Bakterien über Nacht bei 37°C in geeignetem Medium im Wasserbad inkubiert werden. Die so erhaltene Kultur wird am nächsten Tag als Impfkultur für ein größeres Volumen verwendet. Sobald sich die Bakterien in der logarithmischen Wachstumsphase befinden, was durch Messung der optischen Dichte leicht bestimmt werden kann, werden sie entnommen. Durch Auszählen im Mikroskop stellt man eine Bakteriendichte im Bereich von 1-5 x 10¹⁰ Bakterien pro 10 ml Pufferlösung ein. Zu dieser Bakterienlösung wird bei niedriger Temperatur ca. 0,05-0,5 mg FITC pro 10 ml Carbonatpuffer zugegeben und unter Schütteln inkubiert. Die gefärbten Bakterien werden dann abzentrifugiert und so lange mit Pufferlösung nachgewaschen, bis kein freies FITC im Überstand mehr nachweisbar ist. Die Bakterien können nun entweder in dieser Form verwendet werden, gegebenenfalls vorher lyophilisiert und gelagert werden, wenn neben der Phagozytose-Aktivität auch die Opsonierfähigkeit nachgewiesen werden soll. Für eine andere Ausführungsform des erfindungsgemäßen Verfahrens werden die Bakterien in opsonierter Form verwendet. Dazu werden sie mit humanem Serum, das in der Regel eine Mischung aus Serum verschiedener Probanden ist, bei ca. 35 bis 40°C inkubiert. Anschließend werden die Bakterien abzentrifugiert und mit Pufferlösung gewaschen. Die Bakteriensuspension wird nun auf eine Konzentration im Bereich von ca. 1 bis 10 x 10⁹/ml eingestellt, in Röhrchen dosiert und optional lyophilisiert. Nach dem Lyophilisieren können die tiefgefrorenen Zellen gelagert werden. Mit dieser Präparation ist es möglich, die Menge an markierten Bakterien und deren Fluoreszenzintensität auf einen genau definierten Wert einzustellen. Eine Lagerung bei -20°C in flüssiger Form mit mehrfachem Auftauen und Einfrieren ist ebenfalls möglich.

Eine Bakterien-Präparation, deren Gehalt bekannt ist, wird mit der Probe gemischt, wobei beide Lösungen in etwa dieselbe Temperatur haben sollten. Bei dem erfindungsgemäßen Verfahren wird eine definierte Menge an markierten Bakterien und Probeflüssigkeit verwendet. Dies ist wichtig für die Reproduzierbarkeit und Vergleichbarkeit der erhaltenen Werte und somit eine Grundvoraussetzung für Langzeitstudien und Normalwertangaben. Wenn nur die Phagozytose-Aktivität nachgewiesen werden soll, wird eine E. coli-Präparation verwendet, in der die Bakterien opsoniert sind. Soll auch die Opsonierfähigkeit nachgewiesen werden, so wird eine Präparation verwendet, in der die Bakterien nicht mit Opsoninen beladen sind. Die Vorinkubation zwecks Opsonisierung erfolgt unter Mischen über einen Zeitraum von 5 bis 20 Minuten, bevorzugt 8 bis 15 Minuten. Die Inkubation zur Phagozytose wird bei einer Temperatur von 30 bis 40°C durchgeführt, wobei der Temperaturbereich von 37 bis 40°C bevorzugt ist. Während dieser Zeit lagern die Phagozytose-aktiven Zellen, die im Vollblut vorhanden sind, die opsonierten und markierten Bakterien-Zellen an und beginnen sie aufzunehmen. Nach dem angegebenen Zeitpunkt wird die Phagozytose gestoppt, indem die Mischung abgekühlt wird, bevorzugt auf einen Bereich von 0 bis 10°C. Um in die Zelle aufgenommene Bakterien von außen an der Zelle anliegenden absorbierten Partikeln zu unterscheiden, wird dann eine Substanz zugefügt, die die Fluoreszenz auslöschen kann. Solche als Quencher bezeichnete Substanzen sind bekannt. Bevorzugt wird erfindungsgemäß hierzu Coomassie, Trypanblau, Evansblue oder Kristallviolett verwendet.

Anschließend an diese Behandlung wird zu der Mischung eine Lösung zugegeben, die die in der Lösung vorhandenen Erythrozyten zerstört, die nachzuweisenden Leukozyten jedoch praktisch unversehrt läßt. Hierzu wird bevorzugt eine hochsalzhaltige Lösung verwendet, z.B. eine Lösung, die einen hohen Prozentsatz an Ammoniumchlorid und einen Anteil EDTA enthält. Geeignet zur Zerstörung der Erythrozyten sind auch Lösungen, die Formaldehyd und/oder Diethylenglykol enthalten. Besonders bevorzugt wird eine Lösung verwendet, die als FACS Lysing Solution im Handel ist.

Bei der Lyse-Behandlung kann eine Schrumpfung der Zellen auftreten, wodurch die zu phagozytierenden Partikel und vor allem deren Aggregate im Streulichtbild stören. Um diese Störung auszuschließen, wird daher der Mischung nach der Lyse ein fluoreszierender DNA-Farbstoff Zugesetzt. Da der DNA-Gehalt der Bakterienaggregate deutlich geringer ist als der der Leukozyten, lassen sich diese beiden Zellarten gut unterscheiden. Als DNA-Farbstoff wird ein hierzu bekannter Farbstoff verwendet, der eine andere Wellenlänge hat als der für die Fluoreszenzmarkierung der Bakterien verwendete. Bevorzugt wird hierzu Propidiumjodid verwendet. Bei der Auswertung der Fluoreszenz werden dann nur solche Zellen erfaßt, deren DNA-Gehalt mindestens der einer menschlichen Zelle ist, was Bakterienaggregate, Zellbruchstücke oder Aggregations-Artefakte ausschließt.

Es hat sich als vorteilhaft erwiesen, den Farbstoff- und Bakterienlösungen noch ein Konservierungsmittel zuzusetzen. Bevorzugt wird hierfür Thimerosal verwendet. Die Konzentration des Mittels wird so eingestellt, daß eine ausreichende konservierende Wirkung gegeben ist. Vorteilhaft ist eine Endkonzentration im Bereich von 0,005 bis 0,02 %.

In einer bevorzugten Ausführungsform des erfindungsgemäßen Verfahrens wird weiterhin ein Bakteriostatikum verwendet. Es wurde gefunden, daß bei Einsatz eines Bakteriostatikums eine schmalere Größen- und Fluoreszenzverteilung der Bakterien und damit eine höhere Präzision des Tests erreicht wird. Geeignet sind die auf diesem Gebiet verwendeten Mittel. Bevorzugt wird Chloramphanicol oder Rifampicin eingesetzt.

Anschließend wird dann die Fluoreszenz in einem Fluoreszenz-Analyse-Zytometer, z.B. einem Durchflußzytometer oder einem Bildanalysesystem in an sich bekannter Weise gemessen. Man erhält dabei den Anteil der Leukozyten, die überhaupt Bakterien-Zellen aufgenommen haben und kann darüber die Phagozytose-Aktivität, d.h. die Anzahl der aufgenommenen Bakterien pro Leukozyt, bestimmen. Als Besonderheit wird bei der Erfindung bei der Auswertung der Daten in der Software ein Gate auf die Zellen gesetzt, welche mindestens den eukaryonten DNA-Gehalt und gleichzeitig die Streulichteigenschaften eines Leukozyten besitzt.

In einer bevorzugten Ausführungsform des erfindungsgemäßen Verfahrens wird gleichzeitig die Opsonierfähigkeit des Vollblutes bestimmt, indem man markierte Bakterien verwendet, die nicht mit Opsoninen beladen sind. Da die Human-Leukozyten nur ein Vielfaches schneller solche Bakterien-Zellen aufnehmen können, die sie als "fremd" erkennen können (die also opsoniert sind) zeigt ein Vergleich der aufgenommenen Bakterien in opsoniertem und nicht opsoniertem Zustand, inwieweit in der Vollblutprobe die Opsonierfähigkeit gegeben ist.

Ein weiterer Gegenstand der Erfindung ist ein Reagenzien-Kit zur Bestimmung der Phagozytose-Aktivität von Säugerleukozyten, das dadurch gekennzeichnet ist, daß es eine definierte Menge an Fluoreszenz-markiertem E. coli, eine Quenchinglösung, einen fluoreszierenden DNA-Farbstoff, eine hoch salzhaltige Lösung sowie übliche Puffer physikalisch getrennt voneinander enthält.

Vorteilhafterweise wird der Reagenzien-Kit konserviert durch Zusatz eines Mittels, das weder fluoresziert, noch quencht, noch für die Zellen toxisch ist. Hierfür geeignete Präparate sind an sich bekannt. Bevorzugt wird zur Konservierung Thimerosal zugesetzt. Die Konzentration des Konservierungsmittels liegt bevorzugt im Bereich von 0,005 bis 0,02 %, bezogen auf die fertige Reagenzmischung.

Erfindungsgemäß wird ein Verfahren zur Verfügung gestellt, mit dem die Phagozytose-Aktivität und die Opsonierbarkeit in standardisierbarer und damit vergleichbarer Form bestimmt werden kann. Durch die genau definierten Parameter und Wachstumsbedingungen für die Bakterien ist die Genauigkeit gegenüber bekannten Verfahren verbessert. Ein besonderer Vorteil liegt darin, daß die Bestimmung in Vollblut erfolgen kann. Dies erlaubt die Analyse der phagozytären Eigenschaften von Zellen auch innerhalb des Lymphozyten-Clusters, die bei anderen Verfahren nicht möglich ist, da bei den üblichen Verfahren diese Zellen verworfen werden.

Die Erfindung wird durch die folgenden Beispiele erläutert:

### Beispiel 1

### Herstellung von mit FITC markierten E. coli Bakterien Material:

1. E. coli K12 (Fa. Sigma Best.Nr. E1)
2. FITC (Fa. Sigma Best.Nr. F-7250)
3. LB-Medium (5g NaCl, 5g Hefeextrakt, 10 g Trypton/Liter, pH 7,5)
4. Humanes Serum verschiedener Probanden (gepoolt)
5. Erlenmayerkolben 1000 ml (Fa. Schott)
6. Carbonat-Bicarbonatpuffer, pH 9, 0,9 % NaCl
7. PBS (ohne Ca2+ und Mg2+) (Fa. Biochrom)
8. Schüttelinkubator, (Fa. Infors AG)
9. Beckmann Zentrifuge (j2-21)
10. 50 ml Röhrchen (Fa. Nunc, Best. Nr. 339497
11. UV-Mikroskop (Fa. Olympus BH2)
12. Spektralphotometer (Fa. LKB)

E. coli Bakterien K12 wurden über Nacht bei 37°C in LB-Medium im Wasserbad inkubiert. Die so erhaltene Kultur wurde am nächsten Tag als Impfkultur für ein größeres Volumen LB-Medium verwendet. Nach ca. 2 Stunden befanden sich die Bakterien in der logarithmischen Wachstumskurve, was durch Messung der optischen Dichte festgestellt wurde. Der Bakteriengehalt wurde im Mikroskop ausgezählt in einer Neubauerkammer bei 40facher Vergrößerung. Es wurden dann pro 2 x 10¹⁰ Bakterien 10 ml Carbonatpuffer mit 0,1 mg FITC zugegeben. Diese Mischung wurde 30 Minuten lang bei 0°C geschüttelt. Die gefärbten Bakterien wurden abzentrifugiert und so oft mit PBS gewaschen, bis kein freies FITC im Überstand mehr sichtbar war, was durch visuelle Kontrolle der Färbung im UV-Mikroskop kontrolliert wurde. Das Bakterienpellet wurde dann 30 Minuten mit einer Mischung humaner Seren von 7 Probanden behandelt. 8 x 10¹¹ Bakterien wurden mit 50 ml dieses Serums bei 37°C unter Schütteln inkubiert, wobei die Bakterien opsoniert wurden. Nach dem Abzentrifugieren der Bakterien wurden diese zweimal mit kaltem PBS gewaschen. Die Bakterien-Suspension wurde dann auf einen Gehalt von 2 x 10⁹ pro ml eingestellt und es wurde jeweils 1 ml dieser Suspension in ein Reagenzgläschen dosiert und anschließend lyophilisiert. Nach dem Lyophilisieren wurden die Zellen trocken bei -20°C im Dunkeln gelagert.

### Beispiel 2

Bestimmung der Phagozytose-Aktivität von Monozyten und Granulozyten im Vollblut.

### Materialien:

1. FITC-markierte E. coli, hergestellt wie in Beispiel 1 beschrieben = Opsonisierte und FITC markierte E. coli (gemäß SAP Nr. 11/89-001)
2. 5 ml Röhrchen (Sarstedt Best. Nr. 62-476)
3. Vortex (Vortex Genie, Fa. Bender & Hobein, Modell K 550-GE)
4. Hermle Zentrifuge ZK 364
5. Schüttelwasserbad
6. Digitales Thermometer (Fa. Schott)
7. Trypanblau 0,3 % (Fa. Sigma, Best.Nr. T 6146)
8. Propidiumiodid (PI, Sigma P4170, 4 mg/ml in Dimethylformamid oder PBS = 100 x Stammlösung)
9. PBS (Phosphate buffered saline ohne Ca2++ und Mg2++) mit 0,02 % EDTA
10. FACS-Lysing-Solution 10 x (Fa. Becton Dickinson Best.Nr. 92 0002)
11. FACScan Durchflußzytometer (Fa. Becton Dickinson)
12. Calibrite Eichpartikel (Fa. Becton Dickinson, Best.Nr. 95-0002)
13. Dispenser mit Dispenserspritzen (Fa. Eppendorf)

Frisch entnommenes, heparinisiertes Vollblut wurde aufgeschüttelt und in 5 ml Röhrchen dosiert, wobei pro Ansatz 100 »l verwendet wurden. Die Blutproben wurden vor der Zugabe der Bakterien ungefähr 15 Minuten auf Eis inkubiert, um sie auf 0 bis 4°C abzukühlen. Die E. coli Bakterien-Suspension (SAP8/88-001, 1 x 10⁹/ml) wurde gut aufgeschüttelt und 20 »l pro Ansatz zum Vollblut pipettiert. Alle Ansätze wurden geschüttelt. Die Kontrollen blieben auf Eis stehen, die Phagozytose-Ansätze wurden 10 Minuten bei 37°C im Wasserbad unter starkem Schütteln inkubiert. Nach 10 Minuten wurden alle Proben zum Abstoppen der Phagozytose aus dem Wasserbad in Eiswasser gestellt und je 100 »l eiskaltes Trypanblau zu allen Ansätzen zupipettiert. Je Ansatz wurden 2 ml eiskaltes PBS/0,02 % EDTA zugegeben und dann 10 Minuten bei 900 Upm abzentrifugiert. Das Pellet wurde dann noch einmal mit PBS gewaschen. Es wurde dann 3 ml FACS-Lysing-Solution zugegeben und bei Raumtemperatur 5 Minuten inkubiert und anschließend bei Raumtemperatur 5 Minuten mit 1000 Upm abzentrifugiert. Die Proben wurden dann mit kaltem PBS gewaschen und mit 10 »l Propidiumjodid-Lösung 10 Minuten inkubiert. Anschließend erfolgte die Auswertung im Durchflußzytometer (FACscan) unter Verwendung von handelsüblicher (z.B. Consort 30)-Software. Dabei wurde ein sogenanntes Live-Gate im Histogramm der Rotfluoreszenz (Fluoreszenz von Propidiumjodid) auf die Ereignisse gesetzt, die mindestens den DNA-Gehalt einer menschlichen Zelle besitzen, wodurch die Auswertung von Bakterien-Aggregaten ausgeschlossen wurde. Die Fluoreszenz-Verstärkung des FACScan wurde jeden Tag mit frischen kalibrierten Eichpartikeln (unmarkiert und mit FITC markiert) standardisiert und kontrolliert durch Zweipunkt-Messung. Ausgewertet wurde der prozentuale Anteil der phagozytierenden Zellen (dentritische Zellen, Monozyten und Granulozyten) sowie deren mittlere Fluoreszenz-Intensität (Anzahl der phagozytierten Bakterien pro Leukozyt). Dazu wurde das entsprechende Leukozytencluster im Consort 30-Programm im Streulichtdiagramm (lin FSC/y gegen lin SSC/x) gegated und dessen Histogramm der Grünfluoreszenz (Fluoreszenz von FITC) ausgewertet. Dazu wurde eine Region auf die gegenüber der Eiswasser-Kontrolle (Inkubation der Blutprobe mit Bakterien bei 0°C) positiven grünfluoreszenten Zellen gesetzt und deren mittlere Fluoreszenz-Intensität abgelesen, was die mittlere Anzahl der Bakterien pro einzelnem Leukozyt ergibt.

FACS Lysing Solution, FACScan™ und Consort 30™ sind Markennamen der Fa. Becton Dickinson.

## Patentansprüche

1. Verfahren zur Bestimmung der Phagozytose-Aktivität von Säugerleukozyten, **dadurch gekennzeichnet,** daß man eine Körperflüssigkeit mit einer definierten Menge von Fluoreszenz-markierten Bakterien versetzt, unter Schütteln 5 bis 60 Minuten bei einer Temperatur von 30 bis 40°C inkubiert, die Phagozytose durch Abkühlen stoppt, eine guenchende Substanz zugibt, die in der Lösung vorhandenen, nicht phagozytierten Bakterien in ihrer Fluoreszenz unterdrückt, eine Salzlösung zugibt, die die in der Probelösung vorhandenen Erythrozyten lysiert, einen fluoreszierenden DNA-Farbstoff zugibt, der ein anderes Emissionsspektrum aufweist als die fluoreszenzmarkierten Bakterien, und anschließend die Fluoreszenz in einem Fluoreszenz-Zytometer auswertet.

2. Verfahren nach Anspruch 1, **dadurch gekennzeichnet,** daß man als Bakterien E. coli, St. aureus oder Pseudomonas aeruginosa verwendet.

3. Verfahren nach Anspruch 1 oder 2, **dadurch gekennzeichnet,** daß man als Fluoreszenz-markierte Bakterien mit FITC markierte E. coli verwendet.

4. Verfahren nach Anspruch 1, **dadurch gekennzeichnet,** daß man die Phagozytose-Aktivität in Vollblut bestimmt.

5. Verfahren nach einem der vorhergehenden Ansprüche, **dadurch gekennzeichnet,** daß man der Probe 1 bis 5 x 10⁹ Bakterien pro ml Reagenzlösung zusetzt.

6. Verfahren nach einem der vorhergehenden Ansprüche, **dadurch gekennzeichnet,** daß man bei einer Temperatur von 35 bis 39°C inkubiert.

7. Verfahren nach einem der vorhergehenden Ansprüche, **dadurch gekennzeichnet,** daß man als quenchende Substanz Coomassie, Trypanblau, Evansblue oder Kristallviolett verwendet.

8. Verfahren nach einem der vorhergehenden Ansprüche, **dadurch gekennzeichnet,** daß als fluoreszierender DNA-Farbstoff Propidiumjodid verwendet wird.

9. Verfahren nach einem der vorhergehenden Ansprüche, **dadurch gekennzeichnet,** daß man mit FITC markierte Bakterien verwendet, die keine Opsonine tragen.

10. Verfahren nach einem der vorhergehenden Ansprüche, **dadurch gekennzeichnet,** daß man die Reagenzlösungen mit Thimerosal konserviert.

11. Verfahren nach einem der vorhergehenden Ansprüche, **dadurch gekennzeichnet,** daß man der Lösung zusätzlich ein Bakteriostatikum zusetzt.

12. Verfahren nach Anspruch 11, **dadurch gekennzeichnet,** daß man als Bakteriostatikum Chloramphenikol verwendet.

13. Verfahren nach einem der vorhergehenden Ansprüche, **dadurch gekennzeichnet,** daß in einem Parallelansatz die Phagozytose-Aktivitäten einmal mit opsonisierten und einmal mit nicht opsonisierten fluoreszenzmarkierten Bakterien bestimmt werden, wobei die Differenz der beiden erhaltenen Werte ein Maß für die Opsonisierungsfähigkeit von Körperflüssigkeiten darstellt.

14. Reagenzien-Kit zur Bestimmung der Phagozytose-Aktivität von Säugerleukozyten nach dem Verfahren von Anspruch 1, **dadurch gekennzeichnet,** daß es eine definierte Menge an Fluoreszenz-markierten Bakterien, eine Quenchinglösung, einen fluoreszierenden DNA-Farbstoff, eine hoch salzhaltige Lösung sowie übliche Puffer physikalisch getrennt voneinander enthält.

15. Reagenzien-Kit nach Anspruch 14, **dadurch gekennzeichnet,** daß die Reagenzlösungen ein Konservierungsmittel, insbesondere Thimerosal, enthalten.

16. Reagenzien-Kit nach Anspruch 14 oder 15, **dadurch gekennzeichnet,** daß die Reagenzienlösungen zusätzlich ein Bakteriostatikum enthalten.

## Claims

1. A method of determining the phagocytic activity of mammalian leucocytes, characterised in that a body fluid is mixed with a given amount of fluorescence-labelled bacteria, is incubated while being agitated for 5 to 60 minutes at a temperature of 30 to 40°C, the phagocytosis is stopped by cooling, a quenching substance is added, the non-phagocytised bacteria present in the solution are suppressed it its fluorescence, a salt solution is added which causes the erythrocytes present in the sample solution to undergo lysis, a fluorescent DNA dye is added which has a different emission spectrum to the fluorescence-labelled bacteria and, subsequently, the fluorescence evaluated in a fluorescence cytometer.

2. A method according to Claim 1, characterised in that E. coli, St. aureus or Pseudomonas aeruginosa are used as bacteria.

3. A method according to Claim 1 or 2, characterised in that E. coli labelled with FITC are used as fluorescence-labelled bacteria.

4. A method according to Claim 1, characterised in that the phagocytic activity in whole blood is determined.

5. A method according to any one of the preceding Claims, characterised in that 1 to 5 x 10⁹ bacteria per ml of reagent solution is added to the sample.

6. A method according to any one of the preceding Claims, characterised in that incubation is carried out at a temperature of from 35 to 39°C.

7. A method according to any one of the preceding Claims, characterised in that Coomassie, trypan blue, Evans blue or crystal violet is used as the quenching substance.

8. A method according to any one of the preceding Claims, characterised in that propidium iodide is used as the fluorescent DNA dye.

9. A method according to any one of the preceding Claims, characterised in that bacteria labelled with FITC and having no opsonins are used.

10. A method according to any one of the preceding Claims, characterised in that the reagent solutions are preserved with thimerosal.

11. A method according to any one of the preceding Claims, characterised in that a bacteriostatic is additionally added to the solution.

12. A method according to Claim 11, characterised in that chloramphenicol is used as the bacteriostatic.

13. A method according to any one of the preceding Claims, characterised in that in a parallel batch the the phagocytic activities are determined, on the one hand, with opsonised fluorescence-labelled bacteria and, on the other hand, with non-opsonised fluorescence-labelled bacteria, the difference in the two values obtained representing an indication of the opsonising capacity of body fluids.

14. A reagents kit for determining the phagocytic activity of mammalian leucocytes according to the method of Claim 1, characterised in that it contains, physically separated from one another, a given amount of fluorescence-labelled bacteria, a quenching solution, a fluorescent DNA dye, a solution with a high salt content and conventional buffers.

15. A reagents kit according to Claim 14, characterised in that the reagent solutions contain a preservative, in particular thimerosal.

16. A reagents kit according to Claim 14 or 15, characterised in that the reagent solutions additionally contain a bacteriostatic.

## Revendications

1. Procédé pour déterminer l'activité de phagocytose de leucocytes de mammifère, caractérisé en ce que l'on ajoute à un liquide biologique une quantité définie de bactéries marquées par fluorescence, on incube sous agitation pendant 5 à 60 minutes à une température de 30 à 40°C, on arrête la phagocytose par refroidissement, on ajoute une substance extinctrice qui réprime les bactéries non phagocytées présentes dans la solution en ce qui concerne leur fluorescence, on ajoute une solution saline qui lyse les érythrocytes présents dans la solution échantillon, on ajoute un colorant d'ADN fluorescent qui présente un autre spectre d'émission que les bactéries marquées par fluorescence puis on évalue la fluorescence dans un cytomètre à fluorescence.

2. Procédé selon la revendication 1, caractérisé en ce que l'on utilise comme bactéries E. coli, St. aureus ou Pseudomonas aeruginosa.

3. Procédé selon la revendication 1 ou 2, caractérisé en ce que l'on utilise comme bactéries marquées par fluorescence E. coli marquées avec FITC.

4. Procédé selon la revendication 1, caractérisé en ce que l'on détermine l'activité de phagocytose dans le sang total.

5. Procédé selon l'une des revendications précédentes, caractérisé en ce que l'on ajoute à l'échantillon 1 à 5 x 10⁹ bactéries par ml de solution de réactifs.

6. Procédé selon l'une des revendications précédentes, caractérisé en ce que l'on incube à une température de 35 à 39°C.

7. Procédé selon l'une des revendications précédentes, caractérisé en ce que l'on utilise comme substance extinctrice le bleu de Coomassie, le bleu trypane, le bleu d'Evans ou le violet de méthyle.

8. Procédé selon l'une des revendications précédentes, caractérisé en ce que l'on utilise comme colorant d'ADN fluorescent l'iodure de propidium.

9. Procédé selon l'une des revendications précédentes, caractérisé en ce que l'on utilise des bactéries marquées avec FITC qui ne portent aucune opsonine.

10. Procédé selon l'une des revendications précédentes, caractérisé en ce que l'on conserve les solutions de réactifs avec du thimérosal.

11. Procédé selon l'une des revendications précédentes, caractérisé en ce que l'on ajoute en plus à la solution un bactériostatique.

12. Procédé selon la revendication 11, caractérisé en ce que l'on utilise le chloramphénicol comme bactériostatique.

13. Procédé selon l'une des revendications précédentes, caractérisé en ce que, dans une charge parallèle, les activités de phagocytose sont déterminées d'une part avec des bactéries marquées par fluorescence opsonisées et d'autre part avec des bactéries marquées par fluorescence non opsonisées, la différence des deux valeurs obtenues constituant une mesure de la capacité d'opsonisation des liquides corporels.

14. Trousse de réactifs pour la détermination de l'activité de phagocytose de leucocytes de mammifère selon le procédé de la revendication 1, caractérisée en ce qu'elle contient, physiquement séparés les uns des autres, une quantité définie de bactéries marquées par fluorescence, une solution extinctrice, un colorant d'ADN fluorescent, une solution à haute teneur en sel et des tampons habituels.

15. Trousse de réactifs selon la revendication 14, caractérisée en ce que les solutions de réactifs contiennent un conservateur, en particulier du thimérosal.

16. Trousse de réactifs selon la revendication 14 ou 15, caractérisée en ce que les solutions de réactifs contiennent en outre un bactériostatique.
